# EUROPEAN PATENT APPLICATION

(11) **EP 4 249 482 A1**
(43) Date of publication of application: **27.09.2023**
(21) Application number: 21911508.6
(22) Date of filing: 22.12.2021
(51) Int. Cl.: C07D 403/06, A61K 31/5377, A61P 3/04, A61P 3/10, A61P 29/00, A61P 15/10

(54) **AMORPHOUS MELANOCORTIN RECEPTOR AGONIST AND METHOD FOR PREPARING SAME**

(30) Priority: 22.12.2020 KR 20200180806
(71) Applicant: Lg Chem, Ltd., Seoul 07336 (KR)
(72) Inventor: KIM, Ji Yoon, Daejeon 34122 (KR); KANG, Seung Wan, Daejeon 34122 (KR); CHUN, Seul Ah, Daejeon 34122 (KR); KIM, Sung Won, Daejeon 34122 (KR)
(74) Representative: Hoffmann Eitle
(86) International application number: PCT/KR2021/019581
(87) International publication number: WO 2022/139441

(57) **Abstract**

The present invention relates to an amorphous compound represented by chemical formula 1, a method for preparing same, and a pharmaceutical composition comprising same. The amorphous compound represented by chemical formula 1 of the present invention may be characterized by the XPRD pattern, the DSC profile, and/or the NMR spectrum.

## Description

### TECHNICAL FIELD

### Cross-reference to Related Applications

This application claims the benefit of priority based on Korean Patent Application No. 10-2020-0180806, filed on 22 December 2020, the entire disclosure of which is incorporated as part of the specification.

### Technical Field

The present invention relates to a novel amorphous compound exhibiting an excellent agonistic activity for a melanocortin receptor, a method for preparing the same, and a pharmaceutical composition comprising the same.

### BACKGROUND ART

Leptin protein is a hormone secreted by adipocytes, and its secretion amount increases with an increase in body fat content. It regulates functions of various neuropeptides produced from hypothalamus, thereby regulating various in vivo functions, including appetite, body fat content, and energy metabolism (Schwartz, et al., Nature 404, 661-671 (2000)). The leptin protein signal transduction for controlling appetite and body weight is made through the regulation of many factors downstream, the most representative of which are melanocortin, agouti-related peptide (AgRP), and neuropeptide Y (NPY) hormones.

When the concentration of leptin in the blood increases as a result of excess calories in vivo, the secretion of proopiomelanocortin (POMC) protein hormone from the pituitary gland increases and the production of AgRP and NPY decreases. A small peptide hormone, alpha-melanocyte-stimulating hormone (MSH), is produced from POMC neurons. The hormone is an agonist for melanocortin-4 receptors (MC4R) of second-order neurons and ultimately induces appetite decrease. Meanwhile, when the concentration of leptin decreases as a result of calorie deficiency, the expression of AgRP, an MC4R antagonist, increases, and the expression of NPY also increases, which ultimately promotes appetite. That is, according to the change of leptin, the alpha-MSH hormone and the AgRP hormone act as agonists and antagonists for MC4R and thus are involved in appetite control.

The Alpha-MSH hormone binds to three MCR subtypes in addition to MC4R to induce various physiological reactions. Five MCR subtypes have been identified so far. Among them, MC1R is expressed mainly in skin cells and is involved in regulating melanin pigmentation (skin pigmentation). MC2R is expressed mainly in the adrenal gland and is known to be involved in the production of glucocorticoid hormones. Its ligand is only adrenocorticotropic hormone (ACTH) derived from POMC. MC3R and MC4R, which are expressed mainly in the central nervous system, are involved in regulating appetite, energy metabolism, and body fat storage efficiency, and MC5R expressed in various tissues is known to regulate exocrine function (Wikberg, et al., Pharm Res 42 (5) 393-420 (2000)). In particular, activation of the MC4R receptor induce appetite decrease and energy metabolism increase and thus has an effect of efficiently reducing body weight. Therefore, it has been proven to be a major action point in the development of anti-obesity drugs (Review: Wikberg, Eur. J. Pharmacol 375, 295-310 (1999)); Wikberg, et al., Pharm Res 42 (5) 393-420 (2000); Douglas et al., Eur J Pharm 450, 93-109 (2002); O'Rahilly et al., Nature Med 10, 351-352 (2004)).

The role of MC4R in the control of appetite and body weight was primarily demonstrated through an experiment in an animal model of abnormal expression of the agouti protein (agouti mouse). In the case of the Agouti mouse, it was found that due to genetic mutation, the agouti protein was expressed at a high concentration in the central nervous system and acted as an antagonist of MC4R in the hypothalamus to cause obesity (Yen, TT et al., FASEB J. 8, 479-488 (1994); Lu D., et al. Nature 371, 799-802 (1994)). Subsequent research results showed that the agouti-related peptides (AgRP) similar to the actual agouti protein were expressed in hypothalamic nerves, and these are also known to be antagonists for MC4R and be involved in controlling appetite (Shutter, et al., Genes Dev., 11, 593-602 (1997); Ollman, et al. Science 278, 135-138 (1997)).

Intracerebral administration of alpha-MSH, which is an in vivo MC4R agonist, to animals leads to the effect of reducing appetite. When treating the animals with SHU9119 (peptide) or HS014 (peptide), which are MC4R antagonists, it was observed that appetite increased again (Kask et al., Biochem. Biophys. Res. Comm. 245, 90-93 (1998)). In addition, in animal studies using Melanotan II (MTII, Ac-Nle-c[Asp-His-DPhe-Arg-Trp-Lys]-NH2) and the similar agonist thereof, HP228, after intracerebral, intraperitoneal, or subcutaneous administration, efficiencies of inhibiting appetite, reducing body weight, increasing energy metabolism, etc. were found(Thiele T. E., et al. Am J Physiol 274 (1 Pt 2), R248-54 (1998); Lee M. D., et al. FASEB J 12, A552 (1998); Murphy B., et al. J Appl Physiol 89, 273-82 (2000)). On the contrary, administration of the representative SHU9119 to animals showed significant and sustained feed intake and weight gain, providing pharmacological evidence that MCR agonists could be anti-obesity agents. The effect of reducing appetite, which is clearly exhibited upon administration of MTII, was not observed in MC4R knock-out (KO) mice. This experimental result proves again that the appetite-reducing effect is achieved mainly through the activation of MC4R (Marsh, et al., Nat Genet 21, 119-122 (1999)).

Anorectic agents acting on the central nervous system were the main types of antiobestic drugs developed so far. Among them, most were drugs that modulate the action of neurotransmitters. Examples include noradrenalin agents (phentermine and mazindol), serotonergic agents, fluoxetine and sibutramine, and the like. However, the neurotransmitter modulators have a wide range of effects on various physiological actions in addition to appetite suppression, through numerous subtype receptors. Accordingly, the modulators lack selectivity for each subtype, and thus have a major disadvantage in that they are accompanied by various side effects when administered for a long period.

Meanwhile, melanocortin agonists are neuropeptides, not neurotransmitters. Given that in MC4R gene KO mice, all functions other than energy metabolism are normal, they have an advantage as an action point in that they can induce only weight loss through appetite suppression without affecting other physiological functions. In particular, the receptor is a G-protein coupled receptor (GPCR) that belongs to the most successful category of new drug action points developed so far. Thus, the action point greatly differs from existing action points in that it is relatively easy to secure selectivity for subtype receptors.

As an example of utilizing a melanocortin receptor as an action point, international publication nos. WO 2008/007930 and WO 2010/056022 disclose compounds as agonists of the melanocortin receptor.

In addition, the inventors of the present invention have conducted extensive studies and invented a novel compound of the following formula 1 having an excellent agonistic activity selective for a melanocortin receptor, in particular, melanocortin-4 receptor (MC4R), and a method for preparing the same (application no. KR 10-2019-0141649 (filed on 7 November 2019)): 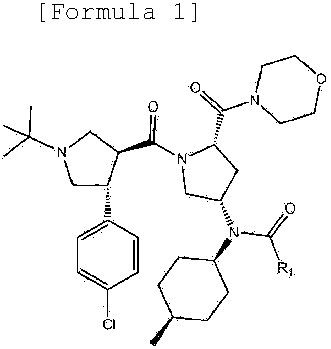 (R₁ is C₂-C₅ alkyl.)

Meanwhile, the salt form or crystal structure of a pharmaceutically active ingredient often affects the chemical stability of the drug. Different salt forms of a compound can change the easiness of filtering the compound, product stability, storage stability, etc. Different crystallization conditions and storage conditions for the compound can lead to changes in the crystal structure of the compound and sometimes the accompanying production of other forms of the crystalline form. Accordingly, it is necessary to study salt forms and/or crystal structures having high purity and good chemical stability for a single compound.

### [Prior art documents]

### [Patent Documents]

(Patent Document 1) International Patent Application Publication No. WO 2008/007930
(Patent Document 2) International Patent Application Publication No. WO 2010/056022

### DISCLOSURE OF THE INVENTION

### TECHNICAL PROBLEM

An objective of the present invention provides a novel amorphous compound having an excellent agonistic activity, which is selective for a melanocortin receptor, in particular, melanocortin-4 receptor (MC4R), and a salt thereof.

Another objective of the present invention provides a method for preparing the amorphous compound or the salt thereof.

Another objective of the present invention provides a pharmaceutical composition comprising the amorphous compound or the salt thereof.

### TECHNICAL SOLUTION

To achieve the above objectives,
According to an aspect of the present invention, there is provided an amorphous compound of the following formula 1: 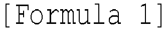 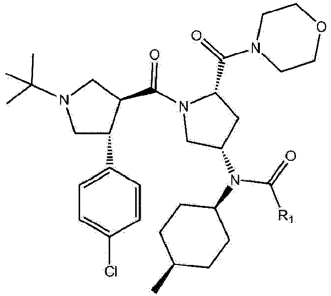 wherein R₁ is C₂-C₅ alkyl.

Since the compound of formula 1 can have an asymmetric carbon center and an asymmetric axis or an asymmetric plane, it can exist as cis or trans isomers, R or S isomers, racemates, diastereomer mixtures, and individual diastereomers, all of which are within the scope of the compound of formula 1.

In the present specification, unless otherwise specified for convenience, the compound of formula 1 is used to include all of the compound of formula 1, a pharmaceutically acceptable salt, an isomer, and a solvate thereof.

In one embodiment, according to the present invention, in formula 1, R₁ is C₂ to C₅ alkyl. In another embodiment, according to the present invention, in formula 1, R₁ is a straight-chain or branched C₂ to C₅ alkyl, for example, ethyl, n-propyl, iso-propyl, n-butyl, iso-butyl, sec-butyl, or tert-butyl.

In another embodiment, according to the present invention, in formula 1, R₁ is C₂ to C₄ alkyl. In another embodiment, according to the present invention, in formula 1, R₁ is a straight-chain or branched C₂ to C₄ alkyl, for example, ethyl, n-propyl, iso-propyl, n-butyl, iso-butyl, sec-butyl, or tert-butyl. Specifically, R₁ may be iso-propyl.

In one embodiment, according to the present invention, the pharmaceutically acceptable salt includes, but is not limited to, acid-addition salts which are formed from inorganic acids, such as hydrochloric acid, sulfuric acid, nitric acid, phosphoric acid, hydrobromic acid, and hydroiodic acid; organic carbonic acids, such as tartaric acid, formic acid, citric acid, acetic acid, trichloroacetic acid, trifluoroacetic acid, gluconic acid, benzoic acid, lactic acid, fumaric acid, and maleic acid; or sulfonic acids, such as methanesulfonic acid, benzenesulfonic acid, p-toluenesulfonic acid, or naphthalene-sulfonic acid.

In one embodiment, according to the present invention, the solvate may include a hydrate; and a solvate with an organic solvent, such as methanol, ethanol, 2-propanol, 1,2-propanediol, 1,3-propanediol, n-butanol, 1,4-butanediol, tert-butanol, acetic acid, acetone, butyl acetate, methyl acetate, ethyl acetate, propyl acetate, t-butyl acetate, isobutyl acetate, methylethylketone, 2-pentanone, tetrahydrofuran, acetonitrile, chloroform, toluene, and mixtures thereof.

The amorphous compound, according to the present invention, may have an X-ray powder diffraction (XRPD) pattern having no characteristic diffraction peaks and having a broad noise.

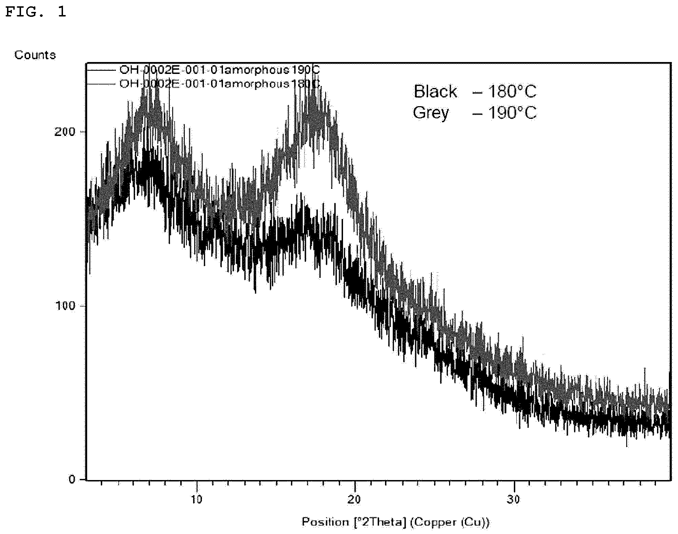

In one embodiment, according to the present invention, the amorphous compound may have the XRPD pattern shown in FIG. 1.

In the hyper differential scanning calorimetry (hyper DSC) profile, the amorphous compound, according to the present invention, may have no exothermic peak when heated to a temperature of 350°C or lower. In addition, in the hyper DSC profile, the amorphous compound is characterized by a glass transition temperature (Tg) of about 98°C.

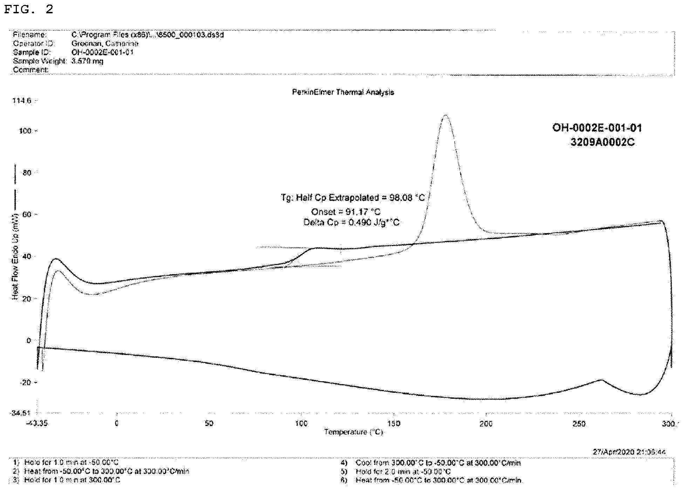

In one embodiment, according to the present invention, the amorphous compound may have the hyper DSC profile shown in FIG. 2.

In the present specification,
X-ray powder diffraction (XRPD) analysis shows the results obtained using PANalytical X' Pert Pro MPD system (Malvern Panalytical Ltd.).

Differential scanning calorimetry (DSC) analysis shows the results obtained using DSC8500 (Perkin Elmer).

Stability analysis shows the results obtained using HPLC (Agilent Technologies, Inc.).

The nuclear magnetic resonance (NMR) spectrum shows the result obtained using Bruker 500 MHz.

The amorphous compound of formula 1 may have higher purity than a crude compound and be physically and chemically more stable.

The amorphous compound of formula 1, according to the present invention, may be formed from a crystalline form I of the compound of formula 1, and the crystalline form I of the compound of formula 1 may have 3 or more, 5 or more, 7 or more, 9 or more, 10 or more, 13 or more, 15 or more, 17 or more, 20 or more, 23 or more, 25 or more, 27 or more, or 30 or more characteristic peaks selected from among peaks with the following diffraction angles (2θ values) of: 8.24010.2°, 9.363±0.2°, 10.2693±0.2°, 10.5969±0.2°, 12.050±0.2°, 12.841±0.2°, 13.503±0.2°, 15.5738±0.2°, 16.6030±0.2°, 17.009±0.2°, 17.305±0.2°, 18.364±0.2°, 18.7390±0.2°, 19.188±0.2°, 19.476±0.2°, 20.001±0.2°, 20.477±0.2°, 20.665±0.2°, 21.348±0.2°, 21.976±0.2°, 22.580±0.2°, 23.896±0.2°, 4.334±0.2°, 24.812±0.2°, 25.243±0.2°, 25.833±0.2°, 26.646±0.2°, 27.82±0.2°, 28.316±0.2°, 28.609±0.2°, 29.692±0.2°, 30.185±0.2°, and 30.875±0.2°in the X-ray powder diffraction (XRPD) pattern.

The amorphous compound of formula 1 prepared from the crystalline form I of the present invention may have higher purity than an amorphous compound of formula 1 formed from other crystalline forms of the compound of formula 1, and may be physically and chemically more stable. In addition, the agonistic ability for the melanocortin-4 receptor and preventive or therapeutic effects on diseases, such as obesity, diabetes, inflammation, erectile dysfunction, or the like, of the amorphous compound of formula 1 of the present invention, can be more excellent than those of known melanocortin-4 receptor agonists. However, the effects of the present invention are not limited thereto.

In another aspect, the present invention provides a method for preparing the amorphous compound of formula 1, comprising a step of melting the compound of formula 1 and then rapidly cooling.

The amorphous compound of formula 1 may be formed using the compound of formula 1.

The compound of formula 1 may be obtained by the preparation method described in the specification of application no. KR 10-2019-0141649 (filed on 7 November 2019) .

The compound used for preparing the amorphous compound of formula 1 of the present invention may include a crystalline form of the compound of formula 1, specifically the crystalline form I of the compound of formula 1.

The crystalline form I of the compound may have 3 or more, 5 or more, 7 or more, 9 or more, 10 or more, 13 or more, 15 or more, 17 or more, 20 or more, 23 or more, 25 or more, 27 or more, or 30 or more characteristic peaks selected from among peaks with 2θ values of: 8.240±0.2°, 9.363±0.2°, 10.2693±0.2°, 10.5969±0.2°, 12.050±0.2°, 12.841±0.2°, 13.503±0.2°, 15.5738±0.2°, 16.6030±0.2°, 17.009±0.2°, 17.305±0.2°, 18.364±0.2°, 18.7390±0.2°, 19.188±0.2°, 19.476±0.2°, 20.001±0.2°, 20.477±0.2°, 20.665±0.2°, 21.348±0.2°, 21.976±0.2°, 22.580±0.2°, 23.896±0.2°, 4.334±0.2°, 24.812±0.2°, 25.243±0.2°, 25.833±0.2°, 26.646±0.2°, 27.82±0.2°, 28.316±0.2°, 28.609±0.2°, 29.692±0.2°, 30.185±0.2°, and 30.875±0.2°in the X-ray powder diffraction (XPRD) pattern.

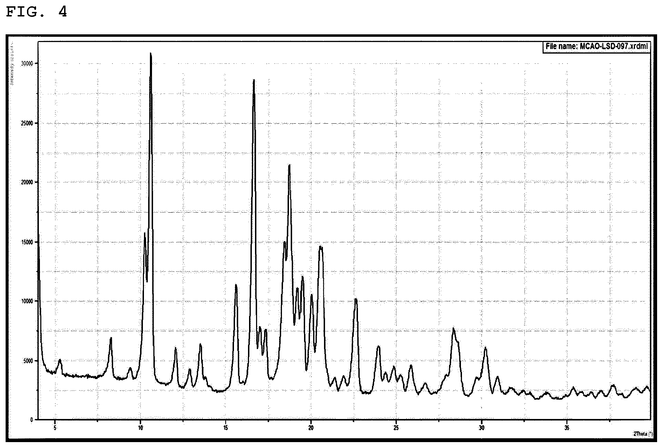

The crystalline form I may have the X-ray powder diffraction (XRPD) pattern shown in FIG. 4.

The amorphous compound of formula 1 of the present invention may be formed by melt quenching method with the compound of formula 1.

First, the compound represented by formula 1 is melted. The melting may be performed under a nitrogen atmosphere.

The melting may be performed at a temperature of, for example, 150°C to 200°C, 160°C to 200°C, 170°C to 190°C, 180°C to 190°C, 180°C, or 190°C.

The melting may be performed for, for example, 30 seconds to 10 minutes, 30 seconds to 5 minutes, 30 seconds to 2 minutes, or 1 minute to 2 minutes.

In one embodiment, according to the present invention, the method may include melting the crystalline form I of the compound of formula 1 for 2 minutes at a temperature of 180°C to 190°C.

Next, the molten compound is rapidly cooled.

The rapid cooling may be performed using liquid nitrogen.

The rapid cooling may be performed by bringing the molten compound of formula 1 into contact with liquid nitrogen.

In one embodiment, according to the present invention, the molten compound of formula 1 may be transferred to a liquid nitrogen bath to form an amorphous compound.

In another embodiment, according to the present invention, liquid nitrogen may be introduced to a flask containing the molten compound to form an amorphous compound.

The amorphous compound of formula 1 as obtained above may have higher purity than a crude compound of formula 1 and may be physically and chemically more stable. However, the effects of the present invention are not limited thereto.

In another aspect, the present invention provides a pharmaceutical composition comprising: (i) the amorphous compound of formula 1 and (ii) a pharmaceutically acceptable carrier.

The amorphous compound of formula 1, according to the present invention, exhibits excellent agonistic actions on melanocortin receptors, in particular, melanocortin-4 receptors (MC4R). Thus, the present invention can provide a pharmaceutical composition for agonizing melanocortin receptors, the composition containing the above-described amorphous compound as an active ingredient. Specifically, the pharmaceutical composition may be a composition for agonizing the function of the melanocortin-4 receptor.

In addition, since the pharmaceutical composition can exhibit excellent effects of preventing or treating obesity, diabetes, inflammation, and erectile dysfunction, it may be a composition for preventing or treating obesity, diabetes, inflammation, or erectile dysfunction. However, the use of the present invention is not limited the diseases.

As used herein, "carrier" refers to a compound that facilitates the introduction of compounds into a cell or tissue.

When the amorphous compound of the present invention is administered for clinical purposes, the total daily dose to be administered to a host in a single dose or in divided doses may be preferably in the range of 0.01 to 10 mg/kg body weight. However, the specific dose level for an individual patient may vary depending on the specific compound to be used, the patient's weight, sex, health status, diet, administration time of the drug, administration method, excretion rate, drug combination, the severity of the disease, or the like.

The amorphous compound of the present invention may be administered by any route as desired. For example, the amorphous compound of the present invention may be administered by injection or orally.

The pharmaceutical composition of the present invention may be in various oral dosage forms, such as tablets, pills, powders, capsules, granules, syrups, or emulsions, or parenteral dosage forms, such as injection preparations for intramuscular, intravenous, or subcutaneous administration.

Preparations for injection may be prepared according to known techniques using suitable dispersing agents, wetting agents, suspending agents, or excipients.

Excipients that can be used in the pharmaceutical preparation of the present invention include, but are not limited to, sweeteners, binders, solubilizers, solubilizing agents, wetting agents, emulsifiers, isotonic agents, adsorbents, disintegrants, antioxidants, preservatives, lubricants, fillers, fragrances, etc. For example, as excipients, lactose, dextrose, sucrose, mannitol, sorbitol, cellulose, glycine, silica, magnesium aluminum silicate, starch, gelatin, gum tragacanth, arginic acid, sodium alginate, methylcellulose, sodium carboxymethyl cellulose, water, ethanol, polyethylene glycol, polyvinyl pyrrolidone, sodium chloride, calcium chloride, orange essence, strawberry essence, vanilla flavor, etc. may be used.

When the pharmaceutical composition of the present invention is in an oral dosage form, examples of the carrier to be used may include, but are not limited to, cellulose, calcium silicate, corn starch, lactose, sucrose, dextrose, calcium phosphate, stearic acid, magnesium stearate, calcium stearate, gelatin, talc, etc.

When the pharmaceutical composition of the present invention is in an injectable preparation form, examples of the carrier may include, but are not limited to, water, saline, aqueous glucose solution, an aqueous sugar-like solution, alcohols, glycol, ethers, oils, fatty acids, fatty acid esters, glycerides, etc.

In another aspect, there is provided an amorphous compound as described above for use in agonizing the functions of melanocortin receptors, in particular, melanocortin-4 receptors (MC4R).

In one embodiment, there is provided an amorphous compound as described above for use in treating or preventing obesity, diabetes, inflammation, or erectile dysfunction.

In another aspect, there is provided a method for agonizing the function of melanocortin receptors, in particular, melanocortin-4 receptors (MC4R), the method comprising a step for administering to a subject the above-described amorphous compound.

In another aspect, there is provided a method for treating obesity, diabetes, inflammation, or erectile dysfunction, the method comprising a step for administering to a subject the above-described amorphous compound.

### ADVANTAGEOUS EFFECTS

The amorphous compound of formula 1, according to the present invention, exhibits excellent agonistic action on melanocortin receptors, in particular, melanocortin-4 receptors (MC4R), and thus can be usefully used for preventing or treating obesity, diabetes, inflammation, and erectile dysfunction.

The amorphous compound of formula 1, according to the present invention, exhibits an on-target effect on melanocortin-4 receptors, thereby exhibiting weight loss and diet reduction effects, without affecting anxiety and depression. In addition, it can be administered without any safety issues, such as side effects of human ether-a-go-go related gene (hERG) inhibition or mutagenesis.

In addition, the amorphous compound of formula 1, according to the present invention, has purity, yield, physical and chemical stability, which are more excellent than the crude compound of formula 1.

Specifically, the amorphous compound of formula 1 may have superior solubility, storage stability, and production stability to the crude compound of formula 1.

### BRIEF DESCRIPTION OF THE DRAWINGS

FIG. 1 is the graph of the XRPD result of Example 1.
FIG. 2 is the graph of the DSC result of Example 1.
FIG. 3 is the graph of the NMR result of Example 1.
FIG. 4 is the graph of the XRPD result of Preparation Example 4.
FIG. 5 is the graph of the DSC result of Preparation Example 4.

### MODE FOR CARRYING OUT THE INVENTION

Hereinafter, the present invention will be described in more detail through Preparation Examples and Examples. However, these Examples are merely illustrative of the present invention, and the scope of the present invention is not limited thereto.

### Preparation Example 1: Preparation of methyl (2S,4S)-4-(N-((1s,4R)-4-methylcyclohexyl)isobutyramido)pyrrolidine-2-carboxylate hydrochloride

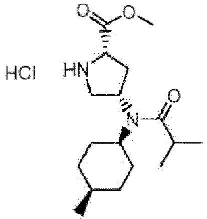

The title compound was obtained through the following steps A, B, C, D, and E.

### Step A: Preparation of 1-(tert-butyl) 2-methyl (2S,4S)-4-azidopyrrolidine-1,2-dicarboxylate

1-(tert-butyl) 2-methyl (2S,4R)-4-((methylsulfonyl)oxy)pyrrolidine-1,2-dicarboxylate (48.5 g, 150 mmol) was dissolved in N,N'-dimethylformamide (250 ml) under nitrogen, and sodium azide (19.5 g, 300 ml) was added. After stirring at 80°C for 16 hours, the reaction solvent was concentrated under reduced pressure, water was added, and extraction was performed twice with ethyl acetate. The organic layer was washed with an aqueous sodium chloride solution and water, dried over anhydrous magnesium sulfate, and filtered. The filtrate was concentrated under reduced pressure to obtain crude 1-(tert-butyl) 2-methyl (2S,4S)-4-azidopyrrolidine-1,2-dicarboxylate (39.59 g, 98%), which was used in the next step without purification.
MS [M+H] = 271 (M+1)
¹H NMR (400 MHz, CD3OD) δ 4.43-4.37 (m, 1H), 4.35-4.27 (br, 1H), 3.77 (s, 1.8H), 3.76 (s, 1.2H), 3.73-3.66 (m, 1H), 3.44-3.38 (m, 1H), 2.63-2.49 (m, 1H), 2.19-2.11 (m, 1H), 1.50 (s, 4.5H), 1.44 (s, 4.5H)

### Step B: Preparation of 1-(tert-butyl) 2-methyl (2S,4S)-4-aminopyrrolidine-1,2-dicarboxylate

1-(tert-butyl) 2-methyl (2S,4S)-4-azidopyrrolidine-1,2-dicarboxylate (24.59 g, 91.0 mmol) obtained in step A above was dissolved in tetrahydrofuran (180 ml), and 1 M trimethylphosphine tetrahydro solution (109.2 ml, 109.2 mmol) was slowly added at 0°C. After stirring at the same temperature for 1 hour, the mixture was stirred at room temperature for 3 hours. After the reaction solvent was concentrated under reduced pressure, dichloromethane (100 ml) and water (150 ml) were added, and the mixture was stirred for about 30 minutes. The layers were separated and were extracted once more with dichloromethane, and the organic layer was dried over anhydrous magnesium sulfate and was filtered. The filtrate was concentrated under reduced pressure to obtain crude 1-(tert-butyl) 2-methyl (2S,4S)-4-aminopyrrolidine-1,2-dicarboxylate (20.62 g, 93%), which was used in the next step without purification.
MS [M+H] = 245 (M+1)
^{1H} NMR (400 MHz, CD3OD) δ 4.27 (m, 1H), 3.77 (s, 1.8H), 3.76 (s,1.2H), 3.75-3.67 (m, 1H), 3.50-3.42 (m, 1H), 3.22-3.17 (m, 1H), 2.58-2.47 (m,1H), 1.82-1.71 (m, 1H), 1.48 (s, 4.5H), 1.42 (s, 4.5H)

### Step C: Preparation of 1-(tert-butyl) 2-methyl (2S,4S)-4-(((1s,4R)-4-methylcyclohexyl)amino)pyrrolidine-1,2-dicarboxylate

1-(tert-butyl) 2-methyl (2S,4S)-4-aminopyrrolidine-1,2-dicarboxylate (20.62 g, 84.4 mmol) obtained in step B above was dissolved in dichloroethane (150 ml), and 4-methylcyclohexanone (9.5 ml, 101.3 mmol) was added. Sodium triacetoxyborohydride (26.8 g, 126.6 mmol) was added at 0°C, and the mixture was stirred at room temperature for 16 hours. The reaction solvent was concentrated under reduced pressure, water was added, and extraction was performed twice with ethyl acetate. The organic layer was washed with an aqueous sodium chloride solution, dried over anhydrous magnesium sulfate, and filtered. The filtrate was concentrated under reduced pressure and purified by column chromatography to obtain 1-(tert-butyl) 2-methyl (2S,4S)-4-(((1s,4R)-4-methylcyclohexyl)amino)pyrrolidine-1,2-dicarboxylate (22.9 g, 80%) .
MS [M+H] = 341 (M+1)
^{1H} NMR (400 MHz, CD3OD) δ 4.26 (m, 1H), 3.76 (s, 1.8H), 3.75 (s, 1.2H), 3.78-3.71 (m, 1H), 3.49-3.40 (m, 1H), 3.22-3.16 (m, 1H), 2.69-2.60(br, 1H), 2.58-2.46 (m, 1H), 1.87-1.77 (m, 1H), 1.73-1.63 (m, 1H), 1.62-1.35(m, 8H), 1.48 (s, 4.5H), 1.42 (s, 4.5H), 0.96 (d, 3H)

### Step D: Preparation of 1-(tert-butyl) 2-methyl (2S,4S)-4-(N-((1s,4R)-4-methylcyclohexyl)isobutyramido)pyrrolidine-1,2-dicarboxylate

1-(tert-butyl) 2-methyl (2S,4S)-4-(((1s,4R)-4-methylcyclohexyl)amino)pyrrolidine-1,2-dicarboxylate obtained in step C above (37.29 g, 109.5 mmol) was dissolved in dichloromethane (500 ml), triethyl amine (61.1 ml, 438.1 mmol) was added, and then isobutyryl chloride (11.7 ml, 219 mmol) was slowly added at 0°C. After stirring at room temperature for 16 hours, the reaction solvent was concentrated under reduced pressure, an aqueous sodium hydrogen carbonate solution was added, and extraction was performed twice with ethyl acetate. The organic layer was washed with an aqueous sodium chloride solution and water, dried over anhydrous magnesium sulfate, and filtered. The filtrate was concentrated under reduced pressure, and purified by column chromatography to obtain 1-(tert-butyl) 2-methyl (2S,4S)-4-(N-((1s,4R)-4-methylcyclohexyl)isobutyramido)pyrrolidine-1,2-dicarboxylate (38.79 g, 86%).
MS [M+H] = 411 (M+1)
^{1H} NMR (400 MHz, CD3OD) δ 4.27 (m, 1H), 3.76 (s, 1.8H), 3.75 (s, 1.2H), 3.78-3.72 (m, 1H), 3.50-3.41 (m, 1H), 3.33-3.14 (m, 1H), 2.69-2.60 (m, 2H), 2.57-2.43 (m, 1H), 1.87-1.79 (m, 1H), 1.70-1.61 (m, 1H), 1.60-1.32 (m, 8H), 1.47 (s, 4.5H), 1.41 (s, 4.5H), 1.10 (dd, 6H), 0.99 (d, 3H)

### Step E: Preparation of methyl (2S,4S)-4-(N-((1s,4R)-4-methylcyclohexyl)isobutyramido)pyrrolidine-2-carboxylate hydrochloride

1-(tert-butyl) 2-methyl (2S,4S)-4-(N-((1s,4R)-4-methylcyclohexyl)isobutyramido)pyrrolidine-1,2-dicarboxylate (34.0 g, 82.8 mmol) obtained in step D above was dissolved in dichloromethane (200 ml), and a solution of 4 N hydrochloric acid in 1,4-dioxane solution (82.8 ml, 331.3 mmol) was added at 0°C. After stirring at room temperature for 6 hours, the reaction solvent was concentrated under reduced pressure to obtain crude (28.7 g, 99%), which was used in the next step without purification.
MS[M+H] = 311 (M+1)

### Preparation Example 2: Preparation of (3S,4R)-1-(tert-butyl)-4-(4-chlorophenyl)pyrrolidine-3-carboxylic acid

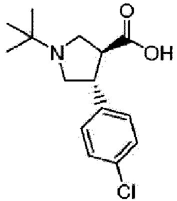

The title compound was obtained according to the method described in international patent publication no. WO 2004/092126.
MS[M+H] = 282 (M+1)
¹H NMR (400 MHz, CD3OD) δ 7.43-7.33 (m, 4H), 3.90-3.69 (m, 3H), 3.59 (dd, J = 11.2, 10.0 Hz, 1H), 3.29 (dd, J = 11.2, 11.2 Hz, 1H), 3.18-3.09 (m, 1H), 1.44 (s, 9H)

### Preparation Example 3: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide (MC70)

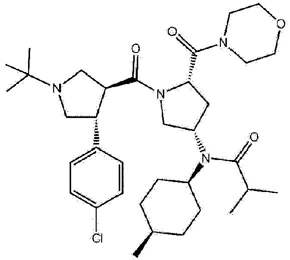

The title compound was obtained through the following steps A, B, and C.

### Step A: Preparation of methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate

Methyl (2S,4S)-4-(N-((1s,4R)-4-methylcyclohexyl)isobutyramido)pyrrolidine-2-carboxylate hydrochloride (28.7 g, 82.73 mmol) obtained in Preparation Example 1, (3S,4R)-1-(tert-butyl)-4-(4-chlorophenyl)pyrrolidine-3-carboxylic acid (24.5 g, 86.87 mmol) obtained in Preparation Example 2, 1-(3-dimethylaminopropyl)-3-ethylcarbodiimide hydrochloride (22.2 g, 115.83 mmol), and 1-hydroxybenzotriazole hydrate (15.7 g, 115.83 mmol) were dissolved in N,N'-dimethylformamide (400 ml), and N,N'-diisopropylethylamine (72.0 ml, 413.66 mmol) was added slowly. After stirring at room temperature for 16 hours, the reaction solvent was concentrated under reduced pressure, 0.5 N sodium hydroxide aqueous solution was added, and extraction was performed twice with ethyl acetate. The organic layer was washed twice with sodium chloride aqueous solution and water, dried over anhydrous magnesium sulfate, and filtered. The filtrate was concentrated under reduced pressure and purified by column chromatography to obtain methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate (41.19 g, 87%).
MS [M+H] = 575 (M+1)

### Step B: Preparation of (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylic acid

Methyl (2S,4S)-1-((3S,4R)-1-(tert-butyl)-4-(4-chlorophenyl)pyrrolidine-3-carbonyl)-4-(N-((1s,4R)-4-methylcyclohexyl)isobutyramido)pyrrolidine-2-carboxylate (39.4 g, 68.62 mmol) obtained in step A above was dissolved in methanol (450 ml), and then, 6 N sodium hydroxide aqueous solution (57.2 ml, 343.09 mmol) was added. After stirring at room temperature for 16 hours, and adjusting the pH to about 5 with 6 N aqueous hydrochloric acid solution, the reaction solution was concentrated under reduced pressure. After dissolving the concentrate in dichloromethane, the insoluble solid was filtered through a paper filter. The filtrate was concentrated under reduced pressure to obtain the crude title compound (38.4 g, 99%), which was used in the next step without purification.
MS [M+H] = 561 (M+1)

### Step C: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide

(2S,4S)-1-((3S,4R)-1-(tert-butyl)-4-(4-chlorophenyl)pyrrolidine-3-carbonyl)-4-(N -((1s,4R)-4-methylcyclohexyl)isobutyramido)pyrrolidine-2-carboxylic acid (38.4 g, 68.60 mmol) obtained in step B above, 1-(3-dimethylaminopropyl)-3-ethylcarbodiimide hydrochloride (18.4 g, 96.04 mmol), and 1-hydroxybenzotriazole hydrate (13.0 g, 96.04 mmol) were dissolved in N,N'-dimethylformamide (200ml), and then morpholine (5.9 ml, 68.80 mmol) and N,N'-diisopropylethylamine (59.7 ml, 343.02 mmol) were sequentially and slowly added. After stirring at room temperature for 16 hours, the reaction solution was concentrated under reduced pressure, 0.5 N sodium hydroxide aqueous solution was added, and extraction was performed twice with ethyl acetate. The organic layer was washed twice with sodium chloride aqueous solution and water, dried over anhydrous magnesium sulfate, and filtered. The filtrate was concentrated under reduced pressure and purified by column chromatography to obtain N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide (37.05 g, 86%, MC70).
MS [M+H] = 630 (M+1)

### Preparation Example 4: Preparation of crystalline form I of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide (the crystalline form I of MC70)

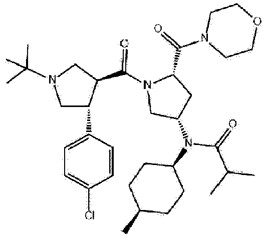

Based on 0.6 g of the compound (MC70) prepared in Preparation Example 3 above, 0.975 mL of MTBE was used to dissolve the compound (MC70) at room temperature for 30 minutes. After dissolution was completed, the mixture was cooled to 3°C and stirred for about 21 hours, and then filtered to obtain the title compound (the crystalline form I of MC70).

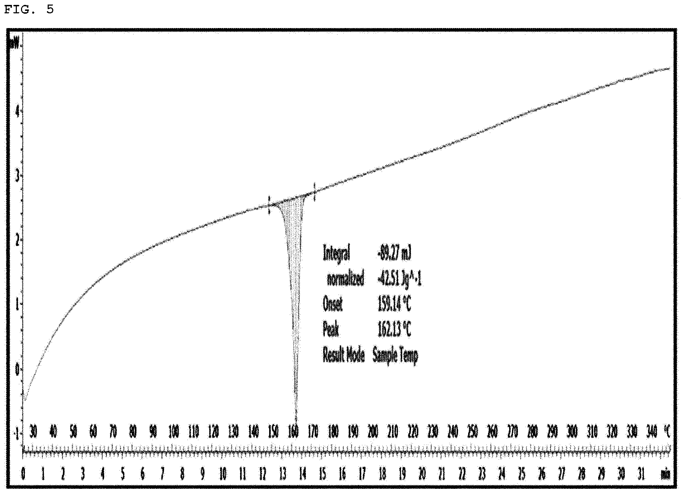

The XRPD (FIG. 4) and DSC (FIG. 5) analyses were performed for the compound of Preparation Example 4 according to the following method. The resulting graphs are shown in FIGs. 4 and 5.

### XRPD Analysis of Preparation Example 4

The XRPD diffraction pattern was obtained using PANalytical X'Pert Pro MPD system equipped with a monochromatized radiation source and Ni filter as a solid-state detector by the following method.

About 20 to 30 mg of the sample was compressed in a glass sample holder so that the sample had a flat surface, the generator of the apparatus was set to 45 kV (acceleration voltage) and 40 mA (filament emission), and then, the measurement was carried out in a reflection mode (not-spin). Bragg angles (2θ) in a range of 4 to 40° were measured with the conditions of step size of 0.026° and time per step of 51 seconds.

### DSC Analysis of Preparation Example 4

The DSC was measured using Mettler Toledo DSC1 system. About 2-5 mg of the sample is weighed and put into a 40 µL Al crucible (flat-bottomed aluminum pan with one pin-hole lid), and one pinhole is made. Then, DSC measurement is performed while the sample is heated from 25°C to 350°C at a rate of 10°C/min. During the measurement, nitrogen gas is supplied to the inside of the instrument at a rate of 70 mL/min to prevent the inflow of oxygen and other gases. Data collection and evaluation were performed using the software STARe.

### Example 1

### Preparation of amorphous compound of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-((1s,4R)-4-methylcyclohexyl)isobutyramide

25 mg of the compound of the crystalline form I prepared in Preparation Example 4 was put into a vial. The vial was filled with nitrogen gas, and then the compound was melted by heating at 180°C or 190°C for 2 minutes. Then, the molten compound was quickly transferred to a liquid nitrogen bath to form an amorphous compound.

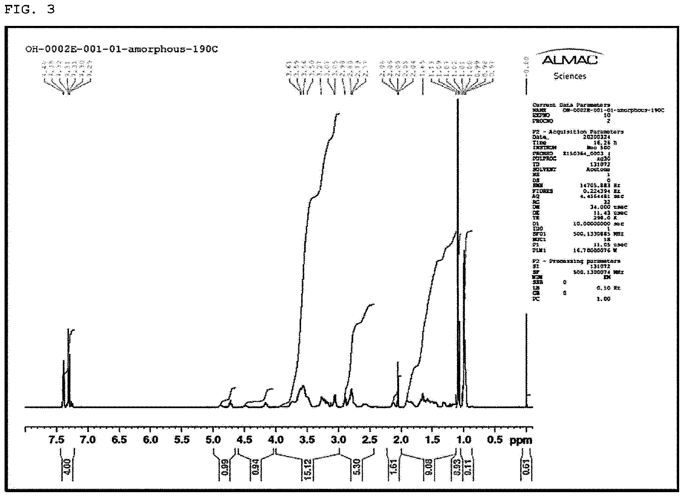

The analysis result of the NMR spectrum of Example 1 (FIG. 3) showed that the compound of Example 1 had the same structure as the compound of Preparation Example 4.

### Amorphous Characteristics

### 1) X-Ray Powder Diffraction (XRPD)

XRPD patterns were obtained by the following method using a Panalytical Xpert Pro diffractometer equipped with a Cu-X-ray tube and a Pixcel detector system.

Samples were analyzed in transmission mode between low-density polyethylene films. Bragg angles (2θ) in a range of 3 to 40° were measured with the conditions of step size of 0.013° and time per step of 22 seconds. The analysis time of the sample was 5 minutes, and the sample was rotated at 60 rpm during data collection. XRPD patterns were classified and processed using HighScorePlus 2.2c software.

The XRPD result is shown in FIG. 1. As shown in FIG. 1, it was confirmed that amorphous Example 1 formed by melting at 180°C and 190°C showed the same pattern. They had no diffraction peaks and exhibited broad noises, typical of amorphous samples.

### 2) Hyper Differential Scanning Calorimetry (Hyper DSC)

DSC was measured using Perkin Elmer DSC8500. Accurately weighed samples were placed in an aluminum sample pan and sealed. Samples were held for 1 minute at -50°C under nitrogen, then heated at a rate of 300°C/min to 300°C, held for 1 minute, cooled back to -50°C, then held for 2 minutes, and then again heated at a rate of 300°C/min to 300°C.

The hyper DSC result is shown in FIG. 2. As shown in FIG. 2, the material of Example 1 exhibits a characteristic having a glass transition temperature at about 98°C, thereby confirming that it exhibits the typical characteristic of an amorphous sample.

### Experimental Example 1. Stability assessment

The following experiments were performed to evaluate the stability of the amorphous compound under various solvent vapours and controlled humidity conditions.

A vapour stress experiment was performed by placing about 25 mg of the amorphous compound of Example 1 into an unsealed vial, placing the vial in a large sealed container containing 1 mL of the selected solvent, and after 5-7 days, analyzing the XRD pattern of the sample. The analysis results are shown in Table 1 below.

In addition, a humidity stress experiment was performed by placing about 25 mg of the amorphous compound of Example 1 in individual vials, storing the vials in an unsealed state in various relative humidity chambers (23% RH, 59% RH, 7% RH, 98% RH, 40°C/75% RH) for 5-7 days, and analyzing the XRD pattern. The analysis results are shown in Table 2 below.

The vapour stress and humidity stress experimental methods are methods used as methods for generating stable solvates or hydrates because, when a substance is exposed to vapours, a solid is plasticized, and molecular mobility is limited. However, for the amorphous compound of Example 1, the amorphous compound was maintained under various conditions according to the above experimental methods. Thus, it was confirmed that the amorphous compound was stable under the corresponding conditions.

**[Table 1]**

| **Input** | **Solvent** | **Result** | **XRD** |
|---|---|---|---|
| Amorphous | Acetone | Solid | Amorphous |
| Amorphous | ACS | Solid | Amorphous |
| Amorphous | Benzyl alcohol | Solid | Amorphous |
| Amorphous | Chlorobenzene | Solid | Amorphous |
| Amorphous | Chloroform | Solid | Amorphous |
| Amorphous | DCM | Solid | Amorphous |
| Amorphous | DME | Solid | Amorphous |
| Amorphous | DMSO | Solid | Amorphous |
| Amorphous | Ethanol | Solid | Amorphous |
| Amorphous | Formamide | Solid | Amorphous |
| Amorphous | Heptane | Solid | Amorphous |
| Amorphous | HFIPA | Solid | Amorphous |
| Amorphous | MeOH | Solid | Amorphous |
| Amorphous | MEK | Solid | Amorphous |
| Amorphous | 2-Me-THF | Solid | Amorphous |
| Amorphous | Nitromethane | Solid | Amorphous |
| Amorphous | NMP | Solid | Amorphous |
| Amorphous | IPA | Solid | Amorphous |
| Amorphous | THF | Solid | Amorphous |
| Amorphous | Water | Solid | Amorphous |
| Amorphous | Pyridine | Solid | Amorphous |

**[Table 2]**

| **Input** | **Screen method** | **Result** | **XRD** |
|---|---|---|---|
| Amorphous | 23% RH stress | Solid | Amorphous |
| Amorphous | 59% RH stress | Solid | Amorphous |
| Amorphous | 75% RH stress | Solid | Amorphous |
| Amorphous | 98% RH stress | Solid | Amorphous |
| Amorphous | 40°C/75% RH stress | Solid | Amorphous |

## Claims

1. An amorphous compound of the following formula 1: 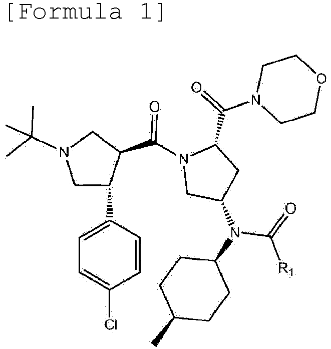 wherein R₁ is C₂-C₅ alkyl.

2. The amorphous compound of formula 1 of claim 1, which has an X-ray powder diffraction (XRPD) pattern having no characteristic diffraction peaks, and having a broad noise.

3. The amorphous compound of formula 1 of claim 1, which has the X-ray powder diffraction pattern shown in FIG. 1.

4. The amorphous compound of formula 1 of claim 1, which has the DSC profile shown in FIG. 2.

5. The amorphous compound of formula 1 of claim 1, which is *N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-((1*s*,4*R*)-4-methylcyclohexyl)isobutyramide.

6. A method for preparing the amorphous compound of formula 1 described in any one of claims 1-5, comprising a step of melting the compound of formula 1 and then rapidly cooling.

7. The preparation method of claim 6, wherein the compound of formula 1 to be melted comprises a crystalline form I of the compound of formula 1, and
the crystalline form I of the compound of formula 1 has an X-ray powder diffraction (XRPD) pattern having 3 or more characteristic peaks selected from among peaks with the following diffraction angles (2θ values) of: 8.24010.2°, 9.363±0.2°, 10.2693±0.2°, 10.5969±0.2°, 12.050±0.2°, 12.841±0.2°, 13.503±0.2°, 15.5738±0.2°, 16.6030±0.2°, 17.009±0.2°, 17.305±0.2°, 18.364±0.2°, 18.7390±0.2°, 19.188±0.2°, 19.476±0.2°, 20.001±0.2°, 20.477±0.2°, 20.665±0.2°, 21.348±0.2°, 21.976±0.2°, 22.580±0.2°, 23.896±0.2°, 4.334±0.2°, 24.812±0.2°, 25.243±0.2°, 25.833±0.2°, 26.646±0.2°, 27.82±0.2°, 28.316±0.2°, 28.609±0.2°, 29.692±0.2°, 30.185±0.2°, and 30.875±0.2°.

8. The method for preparing the amorphous compound of formula 1 of claim 6, wherein the melting is performed at a temperature of 150°C to 200°C.

9. The method for preparing the amorphous compound of formula 1 of claim 6, wherein the melting is performed for 30 seconds to 10 minutes.

10. The method for preparing the amorphous compound of formula 1 of claim 6, wherein the rapid cooling is performed by bringing the molten compound of formula 1 into contact with liquid nitrogen.

11. A pharmaceutical composition comprising the amorphous compound of formula 1 according to any one of claims 1-5 and a pharmaceutically acceptable carrier.

12. A pharmaceutical composition for agonizing the function of a melanocortin-4 receptor, the composition comprising the amorphous compound of formula 1 according to any one of claims 1-5 and a pharmaceutically acceptable carrier.

13. The pharmaceutical composition of claim 12, which is for preventing or treating obesity, diabetes, inflammation, or erectile dysfunction.
